Europäisches Patentamt

European Patent Office

Office européen des brevets

(19)

(11) Veröffentlichungsnummer: **0 029 147**
**B1**

(12) EUROPÄISCHE PATENTSCHRIFT

(45) Veröffentlichungstag der Patentschrift: **28.12.83**

(21) Anmeldenummer: **80106690.3**

(22) Anmeldetag: **30.10.80**

(51) Int. Cl.³: **A 61 F 9/00**

(54) Augentropfenflasche.

(30) Priorität: **16.11.79 DE 2946366**

(43) Veröffentlichungstag der Anmeldung:
**27.05.81 Patentblatt 81/21**

(45) Bekanntmachung des Hinweises auf die Patenterteilung:
**28.12.83 Patentblatt 83/52**

(84) Benannte Vertragsstaaten:
**AT BE CH FR GB IT LI LU NL SE**

(56) Entgegenhaltungen:
**EP - A - 0 013 187**
**US - A - 3 521 636**
**US - A - 4 002 168**
**US - A - 4 134 403**

(73) Patentinhaber: **Basotherm GmbH**
**Postfach 130 Leipzig Strasse 26**
**D-7950 Biberach an der Riss 1 (DE)**

(72) Erfinder: **Hofman-Igl, Ernest**
**Igl Art Haus**
**2285 Kampen/Sylt (DE)**

(74) Vertreter: **Tiedtke, Harro, Dipl.-Ing. et al,**
**Patentanwaltsbüro Tiedtke-Bühling-Kinne- Grupe-**
**Pellmann-Grams Bavariaring 4**
**D-8000 München 2 (DE)**

Courier Press, Leamington Spa, England.

## Augentropfenflasche

Die Erfindung bezieht sich auf eine Augentropfenflasche gemäß Oberbegriff des Patentanspruchs 1.

Es ist eine Augentropfenflasche dieser Gattung bekannt (US—A—3 521 636), bei der der Stützfinger ein scheibenförmiger Vorsprung ist, der am Ende als Stützfläche etwas verbreitert und konkav eingewölbt ist, so daß er auf den Nasenrücken als Stütze aufsetzbar ist. In einem anderen bekannten Fall (US—A—4 134 403) ist an eine Tropfflasche ein gesonderter starrer Stützfinger angeklemmt, der ebenfalls für des Aufsetzen auf den Nasenrücken eine entsprechende konkave Endfläche hat. Beim Ansetzen einer solchen Augentropfenflasche unter Abstützung auf dem Nasenrücken wird die Dosieröffnung in den offenen, wimpernfreien Augenschlitz geführt, so daß keine natürliche Hemmung bewirkt wird, also eine ausreichend genaue Heranführung der Dosieröffnung nicht möglich ist, so daß entweder Fehleinsatz oder auch Verletzungen die Folge sind. Außerdem ist bei dieser Abstützung nur ein kleiner Abschnitt der Augenöffnung mit der Dosieröffnung erreichbar, da der Stützabstand festliegt und Stützfinger und Dosieröffnung in Verlängerung des Auges liegen. Mit Rücksicht auf die Starre Ausbildung des Stützfingers und insbesondere die konkave Auswölbung mit den dadurch bedingten relativ kantigen Enden ist ein anderer Stützort als der Nasenrücken praktisch nicht möglich.

Aufgabe der Erindung ist es, eine Augentropfenflasche gemäß Oberbegriff des Patentanspruch 1 so weiterzubilden, daß eine Dosierung an beliebiger Stelle eines Auges unter exakter Führung bis in unmittelbare Nähe der Hornhaut gewährleistet ist.

Die Aufgabe ist durch die gekennzeichneten Merkmale des Patentanspruchs 1 gelöst. Diese Ausgestaltung ermöglicht es, den Stützfinger oberhalb des Auges abzustützen und von dort in Richtung auf das Auge zu kippen, so daß die Dosieröffnung zwangsläufig an den Augenwimpern vorbei muß, die eine natürliche und sehr empfindliche Hemmung bedeuten und gerade dadurch eine sehr genaue Führung gewährleisten, wobei durch Verschwenken um die Achse des Stützfingers jeder Bereich des Auges über dessen ganze Länge erreicht werden kann. Die Elastizität des Stützfingers gestattet einen schmerzfreien Andruck des Stützfingers, so daß nicht nur der Stützhalt verbessert wird sondern auch unter der elastischen Verformung ein zunehmender federnder Widerstand das Gefühl für die richtige Handhabung fördert.

Weiterbildungen der Erfindung ergeben sich aus den Unteransprüchen.

Die Erfindung wird im folgenden anhand schematischer Zeichnungen an mehreren Ausführungsbeispielen näher erläutert.

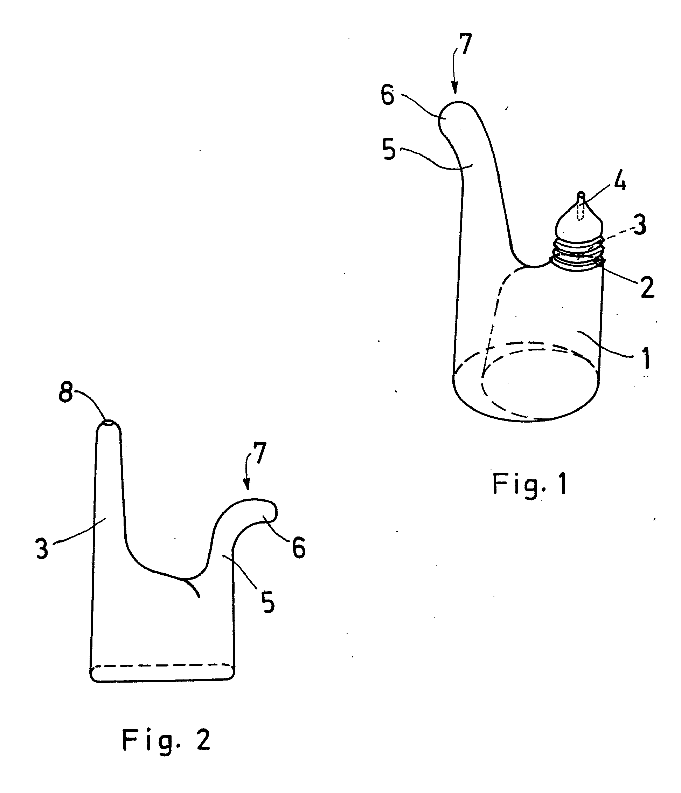

Fig. 1 und 2 zweigen in schaubildlicher Darstellung zwei Ausführungsformen einer erfindungsgemäßen Augentropfenflasche.

Die Augentropfenflasche nach Fig. 1 besitzt einen Korpus 1 aus flexiblem Kunststoff, der im Horizontalschnitt etwa ovale Form hat und auf der Oberseite an einem Ende des Ovals einen Flaschenhals 2 besitzt, dessen Öffnung 3 mit einer Tropfendüse 4 als Dosieröffnung verschließbar ist. Am anderen Ende des Ovals befindet sich ein weichelastisch nachgiebiger Stützfinger 5. Mit Hilfe dieses Stützfingers wird die Flasche am Kopf—z. B. im Bereich der Augenbraue—angesetzt und die Flasche um diesen Stützpunkt unter Heranführen der Tropfendüse an das Auge verschwenkt. Bei seitlichem Druck auf die Flasche kann dann Dosierflüssigkeit an genau wählbarer Stelle in das getropft werden, ohne daß die Düse mit dem Auge in Berührung kommt. Selbst bei unsicherer Hand ist hierdurch die Möglichkeit der Abstützung der Flasche genaue Führung möglich.

Der Stützfinger ist vorzugsweise an seinem freien Ende 6 von der Tropfendüse weggebogen, so daß sich eine weiche Stützfläche 7 ergibt. Der Stützfinger kann mit dem Korpus 1 aus einem Stück bestehen und muß dann so gestaltet sein, daß das abgebogene Ende 6 in Richtung der Abbiegung weichelastisch nachgeben kann. Es kann jedoch der Stützfingerwie gestrichelt angedeutet ist—ein gesonderter, z. B. aus Schaumgummi bestehender Körper sein, der nachträglich angeklebt wird.

Fig. 2 zeigt die Verwirklichung der Erfindung an einer flachen Einwegaugentropfenflasche, die z. B. durch Abquetschen eines Blasformkörpers oder dgl. hergestellt werden kann. In diesem Fall ist kein üblicher Verschluß vorgesehen. Vielmehr ist der Flaschenhals 3 an seinem Ende z. B. durch Einstechen mit einer Öffnung 8 zu versehen.

## Patentansprüche

1. Augentropfenflasche aus elastisch verformbarem Kunststoff, mit einer am Ende eines sich verjüngenden Flaschenhalses (2) befindlichen Dosieröffnung (3, 4) und mit einem Stützfinger (5) im Abstand vom Flaschenhals (2), dadurch gekennzeichnet, daß der Stützfinger (5) aus weichelastischem Werkstoff besteht und zum Zweck der Nachgiebigkeit abgebogen ist.

2. Flasche nach Anspruch 1, dadurch gekennzeichnet, daß der Stützfinger (5) mit der Flasche einstückig verbunden ist.

3. Flasche nach Anspruch 1 oder 2, dadurch gekennzeichnet, daß der Stützfinger (5) kürzer als der Flaschenhals (3) ist.

4. Flasche nach einem der vorhergehenden Ansprüche, dadurch gekennzeichnet, daß Stützfinger (5) und Flaschenhals (3) jeweils symmetrisch zu einer Längsmittelebene der Flasche ausgebildet sind.

5. Flasche nach einem der vorhergehenden Ansprüche, dadurch gekennzeichnet, daß Stützfinger (5) und Flaschenhals (3) etwa V-förmig zueinander stehen.

6. Flasche nach einem der vorhergehenden Ansprüche, dadurch gekennzeichnet, daß sie eine in der Längsmittelebene abgequetschte Einwegflasche ist.

**Revendications**

1. Flacon pour collyre en matière plastique élastiquement déformable, comportant une ouverture de dosage (3, 4) se trouvant à l'extrémité d'un goulot (2) effilé, et un doigt d'appui (5) à distance du goulot, caractérisé en ce que le doigt d'appui (5) est en matière souple et replié pour améliorer le flexibilité.

2. Placon selon la revendication 1, caractérisé en ce que le doigt d'appui (5) est réalisé d'un seul tenant avec le flacon.

3. Flacon selon la revendication 1 ou 2, caractérisé en ce que le doigt d'appui (5) est plus court que le goulot (3).

4. Flacon selon l'une des revendications précédentes, caractérisé en ce que le doigt d'appui (5) et le goulot (3) ont chacun une conformation symétrique par rapport à un plan de symétrie longitudinale du flacon.

5. Flacon selon l'une des revendications précédentes, caractérisé en ce que le doigt d'appui (5) et le goulot (3) sont dans une position relative mutuelle sensiblement en V.

6. Flacon selon l'une des revendications précédentes, caractérisé en ce que c'est un flacon unidirectionnel écrasé dans son plan de symétrie longitudinale.

**Claims**

1. An eyedropper bottle consisting of elastically deformable plastic, having a dispensing opening (3, 4) located at the end of a tapered bottle neck (2) and a supporting finger (5) at a distance from said bottle neck (2), characterized in that said supporting finger (5) consists of soft-elastical material and is bent for the purpose of resilience.

2. A bottle according to claim 1, characterized in that said supporting finger (5) and said bottle are one piece.

3. A bottle according to claims 1 or 2, characterized in that said supporting finger (5) is shorter than said bottle neck (3).

4. A bottle according to one of the preceding claims, characterized in that said supporting finger (5) and said bottle neck (3), each, are formed symmetrically about a longitudinal center plane of said bottle.

5. A bottle according to one of the preceding claims, characterized in that said supporting finger (5) and said bottle neck (3) form an approximate V-shape with one another.

6. A bottle according to one of the preceding claims, characterized in that it is a disposable bottle which is squeezed off in the longitudinal center plane.

Fig. 1

Fig. 2